(19) Europäisches Patentamt
European Patent Office
Office européen des brevets

(11) EP 0 626 363 B1

(12) FASCICULE DE BREVET EUROPEEN

(45) Date de publication et mention de la délivrance du brevet:
**04.03.1998 Bulletin 1998/10**

(51) Int. Cl.[6]: **C07C 45/48**, C07C 49/395

(21) Numéro de dépôt: **94401122.0**

(22) Date de dépôt: **20.05.1994**

# (54) Procédé de préparation d'une cétone cyclique

Verfahren zur Herstellung eines cyclischen Ketons

Process for the préparation of a cyclic ketone

(84) Etats contractants désignés:
**CH DE ES FR GB IT LI**

(30) Priorité: **28.05.1993 FR 9306476**

(43) Date de publication de la demande:
**30.11.1994 Bulletin 1994/48**

(73) Titulaire: **RHONE-POULENC CHIMIE**
**92408 Courbevoie Cédex (FR)**

(72) Inventeurs:
- **Alas, Michel**
  **F-79500 Melle (FR)**
- **Crochemore, Michel**
  **F-69630 Chaponost (FR)**

(74) Mandataire:
**Dutruc-Rosset, Marie-Claude et al**
**RHONE-POULENC CHIMIE,**
**Direction de la Propriété Industrielle,**
**25, Quai Paul Doumer**
**92408 Courbevoie Cédex (FR)**

(56) Documents cités:

| | |
|---|---|
| **EP-A- 0 251 111** | **EP-A- 0 266 687** |
| **EP-A- 0 297 447** | **GB-A- 171 391** |
| **NL-A- 98 127** | **US-A- 2 697 729** |

- **CHEMICAL ABSTRACTS, vol. 81, no. 13, 30 Septembre 1974, Columbus, Ohio, US; abstract no. 77474n, FUKUI, MASAHIRO ET AL. 'Ketones from carboxylic acids.' page 423 ;colonne 1 ; & JP-A-49 030 309 (CHISSO CORP.) 18 Mars 1974**

Remarques:
Le dossier contient des informations techniques présentées postérieurement au dépôt de la demande et ne figurant pas dans le présent fascicule.

EP 0 626 363 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.15.12/3.4

## Description

La présente invention a pour objet un procédé de préparation d'une cétone cyclique. Plus précisément, l'invention concerne un procédé de préparation d'une cétone cyclique, à partir d'un acide dicarboxylique.

L'invention vise plus particulièrement la préparation de la cyclopentanone à partir de l'acide adipique et de la diméthyl-2,2 cyclopentanone à partir de l'acide diméthyl-2,2 adipique.

Il est connu selon GB-A 615 543 de préparer la cyclopentanone, à partir de l'acide adipique, par chauffage de l'acide adipique, en présence de carbonate ou d'oxyde de manganèse. Le rendement obtenu en cyclopentanone, lorsque la cyclisation est effectuée à 280°C, est excellent, de l'ordre de 90 %.
Toutefois, ce procédé ne donne pas entière satisfaction car il n'est pas possible d'accroître la productivité en cyclopentanone.
En effet, pour maintenir les rendements précités, il est nécessaire de limiter les débits d'alimentation en acide adipique à environ 0,7 kg/h, pour un kilogramme de catalyseur engagé.
Le débit d'alimentation pourrait être accru en augmentant la température réactionnelle. Bien que la température mentionnée dans la description puisse être choisie dans une gamme de température allant de 280°C à 350°C, il est très difficile d'un point de vue industriel de maintenir une température supérieure à 320°C, sans équipement onéreux ni fluides calorifiques spéciaux et chers.
De plus, il est très difficile, sinon impossible, de maintenir une telle température de manière homogène, dans un tel milieu visqueux constitué de i'acide adipique liquide et du catalyseur à base de manganèse. En outre, il peut exister en plus des problèmes évoqués précédemment, des risques de dépôt de substances polymériques sur le réacteur (phénomène de blindage) qui sont difficiles à éliminer.

Il est également décrit dans US-A 2 612 524, un procédé de préparation de cyclopentanone par cyclisation de l'acide adipique, en présence d'une catalyseur qui est un oxyde de magnésium. Ce procédé requiert aussi une température élevée puique celle-ci se situe entre 300°C et 350°C.

Selon EP-A-0 251 111, on a décrit la préparation de la cyclopentanone à partir du monoester méthylique ou du diester méthylique de l'acide adipique, en présence d'un catalyseur solide qui est l'oxyde de magnésium, l'alumine, le trioxyde de bore, la silice, l'oxyde de zinc ou l'oxyde de titane

Par ailleurs, on prépare selon EP-A-0 297 447, la 2-cyclopenténone à partir d'acides hexènedioiques ou leurs esters, à des températures de 150°C à 450°C, en présence d'un catalyseur solide du type oxyde, tel que notamment l'oxyde d'aluminium, l'oxyde de magnesium ou le trioxyde de bore.

Par rapport aux procédés décrits dans l'état de la technique, l'objectif de la présente invention est de disposer d'un procédé permettant d'accroître la productivité en cyclopentanone tout en étant aisément mis en oeuvre à l'échelle industrielle, sans faire appel à un appareillage sophistiqué.

Il a maintenant été trouvé et c'est ce qui constitue l'objet de la présente invention, un procédé de préparation d'une cétone cyclique à partir d'un acide dicarboxylique répondant à la formule (I) suivante :

$$HOOC - R - COOH \qquad (I)$$

dans ladite formule (I), R représente un radical alcoylène, substitué ou non, comprenant un enchaînement linéaire d'atomes en un nombre suffisant pour former le cycle cétonique désiré, deux atomes de carbone vicinaux de l'enchaînement du radical R pouvant faire partie d'un cycle carbocyclique ou hététocyclique, saturé ou aromatique.
caractérisé par le fait que l'on conduit la réaction en phase liquide, en présence d'une quantité efficace d'un métal ou dérivé des éléments choisis parmi : le bore, l'aluminium, le gallium, l'indium, le thallium, l'étain, l'antimoine, le bismuth, le molybdène, le rubidium, le césium, le vanadium, à l'exception des phosphates neutres.

Ainsi, les catalyseurs de l'invention et de préférence, les dérivés de l'étain et du bore étant plus actifs que les catalyseurs à base de manganèse, il est possible selon l'invention d'augmenter le débit de l'acide dicarboxylique et plus particulièrement de l'acide adipique, dans un rapport de 1 à 3, voire-même 5.

Conformément au procédé de l'invention, on met en oeuvre un acide dicarboxylique qui répond à la formule (I) dans laquelle R représente un radical alcoylène, substitué ou non, comprenant un enchaînement linéaire d'atomes en un nombre suffisant pour former le cycle cétonique désiré.

Généralement, le radical R comprend un enchaînement linéaire de 2 à 10 atomes et, de préférence, de 2 à 7 atomes et encore plus préférentiellement de 4 ou 5. Il s'agit le plus souvent d'un enchaînement d'atomes de carbone mais l'invention n'exclut pas que la chaîne hydrocarbonée soit interrompue par un hétéroatome, notamment azote, oxygène ou soufre.

Comme mentionné précédemment, le radical R peut être substitué c'est-à-dire que les atomes d'hydrogène de la chaîne hydrocarbonée peuvent être remplacés par un groupe ou une fonction organique. N'importe quels substituants peuvent être présents dans la mesure où ceux-ci n'interfèrent pas au niveau de la réaction de cyclisation. En particulier, la chaîne hydrocarbonée peut porter des chaînes latérales ou ramifications qui peuvent être constituées de préférence

par des radicaux alkyle qui, généralement, ont de 1 à 4 atomes de carbone. Les ramifications se situent le plus souvent sur un ou les deux atomes de carbone en position α ou β des groupes carboxyliques.

D'une manière globale, le radical R présente une condensation totale en carbone pouvant varier largement de 2 atomes de carbone jusqu'à un nombre pouvant s'élever à 40 atomes de carbone lorsqu'il y a présence de substituants et ledit radical comprend un enchaînement linéaire de 2 à 10 atomes qui intervient ensuite dans le cycle obtenu.

Dans la formule (I), R représente, de préférence, un radical alcoylène, linéaire ou ramifié.

Plus précisément, R représente un radical alcoylène linéaire ou ramifié ayant de préférence de 2 à 20 atomes de carbone.

Conviennent tout particulièrement bien à la mise en oeuvre du procédé de l'invention, les acides dicarboxyliques de formule générale (I) dans laquelle le radical R est un radical alcoylène linéaire ou ramifié ayant de 2 à 12 atomes de carbone qui comprend un enchaînement linéaire de 2 à 8 atomes de carbone entre les deux groupes COOH.

Le radical R préféré comprend un enchaînement linéaire de 4 ou 5 atomes de carbone entre les deux groupes COOH.

Il est également possible de faire appel dans le procédé de l'invention, à un acide dicarboxylique de formule (I) dans laquelle deux atomes de carbone vicinaux de l'enchaînement du radical R peuvent former un cycle.

Par cycle, on entend un cycle carbocyclique ou hétérocyclique, saturé ou aromatique.

Comme exemples de cycles, on peut envisager des cycles cycloaliphatiques, aromatiques ou hétérocycliques, notamment cycloalkyles comprenant 6 atomes de carbone dans le cycle ou benzéniques ; ces cycles pouvant être eux-mêmes éventuellement porteurs de un ou plusieurs substituants dans la mesure où ils n'interfèrent pas avec la réaction de cyclisation.

Comme exemples de tels radicaux R, on peut mentionner, entre autres, les radicaux suivants :

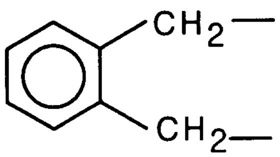

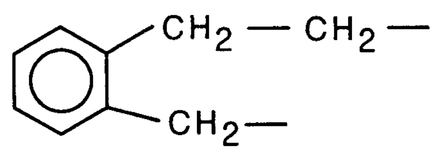

A titre d'acides carboxyliques de formule (I) convenant à la présente invention, on fait appel plus particulièrement aux acides dicarboxyliques suivants :

- l'acide adipique,
- l'acide méthyl-2 adipique
- l'acide méthyl-3 adipique
- l'acide méthyl-4 adipique
- l'acide méthyl-5 adipique
- l'acide diméthyl-2,2 adipique
- l'acide diméthyl-3,3 adipique
- l'acide triméthyl-2,2,5 adipique
- l'acide diméthyl-2,5 adipique
- l'acide pimélique (heptanedioïque)
- l'acide méthyl-2 pimélique
- l'acide diméthyl-2,2 pimélique
- l'acide diméthyl-3,3 pimélique
- l'acide diméthyl-2,5 pimélique
- l'acide triméthyl-2,2,5 pimélique
- l'acide azélaïque
- l'acide sébacique
- l'acide phénylène-1,2 diacétique

Conformément au procédé de l'invention, on effectue la réaction de cyclisation de l'acide dicarboxylique, en présence d'un catalyseur à base de bore ou des éléments métalliques précités : l'aluminium, le gallium, l'indium, le thallium, l'étain, l'antimoine, le bismuth, le molybdène, le rubidium, le césium, le vanadium.

On peut les mettre en oeuvre sous n'importe quelle forme. On peut les apporter sous forme de métal ou d'oxyde ou sous forme saline, sel simple ou double, minéral ou organique.

Le catalyseur mis en oeuvre peut être un catalyseur à base de bore.

A titre de composés de bore, on fait appel, de préférence aux acides boriques tels que l'acide orthoborique (ou son précurseur $B_2O_3$), métaborique, pyro- ou tétraborique ou aux borates métalliques, notamment de métaux alcalins, alcalino-terreux ou d'ammonium sous forme anhydre ou hydratée, en particulier aux tiers borates, hémiborates, monoborates, diborates, triborates, tétraborates, pentaborates de métaux, de préférence alcalins ou d'ammonium.

On peut également faire appel à un sel double contenant du bore, notamment les fluoborates métalliques, par exemple, le fluoborate de potassium.

Comme exemples de composés du bore convenant à l'invention, on peut citer :

- l'acide orthoborique ou son précurseur,
- le métaborate de sodium,
- le métaborate tétrahydraté de sodium,
- le tétraborate de sodium,
- le tétraborate décahydraté de sodium ou borax,
- le tétraborate pentahydraté de sodium,
- le métaborate de potassium,
- le pentaborate tétrahydraté de potassium,
- le tétraborate octahydraté de potassium,
- le pentaborate tétrahydraté d'ammonium,
- le tétraborate tétrahydraté d'ammonium.

D'une manière préférentielle, on fait appel au métaborate de sodium ou de potassium.

Les éléments métalliques précités peuvent être apportés sous forme d'un métal ou sous forme d'un oxyde ou d'un hydroxyde. Il est possible de faire appel à un sel minéral de préférence, nitrate, sulfate, oxysulfate, halogénure, oxyhalogénure, silicate, carbonate, oxalate ; ou à un sel organique de préférence, acétylacétonate, alcoolate et encore plus préférentiellement méthylate ou éthylate, carboxylate et encore plus préférentiellement acétate.

Le catalyseur mis en oeuvre peut être choisi parmi les éléments métalliques du groupe 3b de la Classification périodique des éléments. Il s'agit donc de l'aluminium, du gallium, de l'indium et du thallium.

Pour la définition des éléments, on se réfère ci-après à la Classification périodique des éléments publiée dans le Bulletin de la Société Chimique de France, n°1 (1966).

Concernant l'aluminium, on peut faire appel à l'un des dérivés de l'aluminium et plus particulièrement à l'un des composés cités ci-après :

- le nitrate d'aluminium,
- l'oxalate d'aluminium,
- les oxydes d'aluminium sous forme anhydre $\alpha$ ou $\beta$ ou sous forme de monohydrate ou trihydrate,
- 
- le propoxyde d'aluminium,
- le silicate d'aluminium,
- le sulfate d'aluminium hydraté ou non.

Comme exemples de composés du gallium, on peut mentionner, notamment :

- l'acétate de gallium,
- l'acétylacétonate de gallium,
- les halogénures de gallium et par exemple, le fluorure, chlorure ou bromure de gallium,
- le sesquioxyde de gallium sous forme anhydre ou hydratée,
- le nitrate de gallium,
- le sulfate de gallium sous forme anhydre ou hydratée.

En ce qui concerne les dérivés de l'indium, on choisit préférentiellement l'un des composés suivants :

- les halogénures d'indium et par exemple, le fluorure, chlorure ou bromure d'indium,
- l'hydroxyde d'indium In(OH),
- le monoxyde d'indium,
- le sesquioxyde d'indium sous forme anhydre ou hydratée,
- le nitrate d'indium,
- le sulfate d'indium sous forme anhydre ou hydratée.

Comme exemples de dérivés du thallium, on peut citer, entre autres :

- l'acétate de thallium,
- le carbonate de thallium,
- le méthoxyde de thallium,
- l'éthoxyde de thallium,
- les halogénures de thallium et par exemple, le mono- ou trifluorure, le mono-ou trichlorochlorure ou le mono- ou tribromure de thallium,
- l'hydroxyde de thallium Tl(OH),
- l'oxyde de thallium I ou III,
- le sesquioxyde de thallium sous forme anhydre ou hydratée,
- le nitrate de thallium I ou III,
- l'oxalate de thallium,
- l'hydrogénophosphate de thallium III,
- le sulfate de thallium I ou III,
- l'hydrogénosulfate de thallium II,
- le tartrate de thallium.

En plus des éléments du groupe 3b de la classification periodique, on peut selon l'invention mettre en oeuvre comme catalyseur, les dérivés de l'étain et plus préférentiellement :

- l'acétate d'étain II,
- les halogénures d'étain et par exemple, le fluorure, le chlorure ou le bromure d'étain II ou IV,
- les oxydes d'étain II ou IV sous forme anhydre ou hydratée,
- le nitrate d'étain II ou IV ou le nitrate d'étain II basique,
- le sulfate d'étain II ou IV,
- le stannate de sodium,
- le tartrate d'étain.

Comme exemples de catalyseurs à base d'antimoine, on peut citer notamment :

- les halogénures d'antimoine et par exemple, le fluorure, le chlorure ou le bromure d'antimoine III ou V,
- les oxydes d'antimoine III ou V sous forme anhydre ou hydratée,
- l'oxychlorure d'antimoine III,
- l'oxysulfate d'antimoine III,
- le nitrate d'antimoine basique,
- le sulfate d'antimoine III,
- le tartrate d'antimoine.

Un autre type de catalyseurs convenant à l'invention, sont les dérivés du bismuth et, plus préférentiellement, les composés suivants :

- l'acétate de bismuth I,
- le carbonate de bismuth $Bi_2O_2CO_3$,
- le citrate de bismuth,
- les halogénures de bismuth et par exemple, le triflluorure, le tri- ou tétrachlorure ou le tribromure de bismuth,
- le lactate de bismuth,
- le nitrate de bismuth ou le nitrate de bismuth basique $BiONO_3$, $H_2O$,
- l'oxalate de bismuth,
- l'hydroxyde de bismuth,
- les oxydes de bismuth, en particulier le monoxyde, le trioxyde, le tétraoxyde ou le pentaoxyde de bismuth, sous forme anhydre ou hydratée,
- les oxyhalogénures de bismuth tels que l'oxyfluorure, l'oxychlorure, l'oxyfluorure de bismuth,
- le salicylate basique de bismuth,
- le sulfate de bismuth,
- le tartrate de bismuth.

Comme exemples de catalyseurs à base de molybdène, on peut citer notamment :

- les halogénures de molybdène, par exemple, l'hexafluorure de molybdène, le tri- tétra- ou pentachlorure de molybdène, le di- tri- ou tétrabromure de molybdène,
- les hydroxydes de molybdène $Mo(OH)_3$, $MoO(OH)_3$ ou $Mo_2O_3$, $3H_2O$,
- les oxydes de molybdène tels que le dioxyde, le trioxyde, le pentaoxyde ou le sesquioxyde de molybdène,
- les oxyhalogénures de molybdène tels que l'oxydifluorure ou l'oxytétrafluoure de molybdène, l'oxydichlorure, l'oxytrichlorure, l'oxytétrachlorure, l'oxypentachlorure de molybdène, l'oxychlorure acide de molybdène, l'oxydibromure de molybdène,
- le molybdate d'ammonium.

En ce qui concerne les catalyseurs au rubidium, on peut citer :

- l'acétate de rubidium,
- les halogénures de rubidium et par exemple, le fluorure, le chlorure ou le bromure de rubidium I ou III,
- le carbonate ou le carbonate acide de rubidium,
- l'hydroxyde de rubidium,
- les oxydes de rubidium tels que le monoxyde, le sesquioxyde, le tétraoxyde, le peroxyde de rubidium,
- le nitrate de rubidium,
- le sulfate de rubidium,
- l'hydrogénosulfate de rubidium,
- le tartrate de rubidium.

D'autres catalyseurs susceptibles d'être mis en oeuvre dans le procédé de l'invention sont les catalyseurs à base de césium et plus spécialement les composés suivants :

- l'acétate de césium,
- les halogénures de césium et par exemple, le fluorure, le chlorure ou le bromure de césium,
- les oxydes de césium tels que le trioxyde ou le peroxyde de césium,
- le nitrate de césium basique,
- le nitrate de césium acide,
- le sulfate de césium,
- l'hydrogénosulfate de césium,
- le tartrate de césium.

Conviennent également à la mise en oeuvre du procédé de l'invention, les dérivés du vanadium et plus particulièrement ceux énoncés ci-après :

- les halogénures de vanadium tels que le tri- tétra- ou pentafluorure de vanadium, le di- tri- ou tétrachlorure de vanadium, le tribromure de vanadium,
- les oxydes de vanadium tels que l'oxyde de vanadium, le dioxyde de vanadium, le sesquioxyde de vanadium, le pentaoxyde de vanadium,
- les oxyhalogénures de vanadium en particulier les oxy di- ou trifluorure de vanadium, les oxy mono- di- ou trichlorure de vanadium, les oxy mono-di- ou tribromure de vanadium,
- le sulfate de vanadium,
- le sulfate de vanadyle,
- l'acétylacétonate de vanadyle.

Comme mentionné précédemment, il est possible de faire appel aux sels doubles des éléments tels que définis par l'invention.

A titre d'exemples, on peut citer les sels doubles suivants convenant pour l'invention :

- le sulfate de thallium et d'aluminium $TlAl(SO_4)_2$, $12H_2O$,
- le métavanadate de thallium $TlVO_3$,
- le pyrovanadate de thallium $Tl_4V_2O_7$,
- le molybdate de thallium,
- le vanadate de bismuth,
- le molybdate de bismuth,
- le sulfate d'aluminium et de rubidium $RbAl(SO_4)_2$, $12H_2O$,
- le borofluorure de rubidium,

- le sulfate de vanadium et de rubidium $RbV(SO_4)_2$, $12H_2O$,
- le sulfate d'aluminium et de césium $CsAl(SO_4)_2$, $12H_2O$,
- le borofluorure de césium,
- le sulfate de vanadium et de césium $CsV(SO_4)_2$, $12H_2O$,

Parmi les catalyseurs précités, on choisit préférentiellement ceux qui sont les plus facilement accessibles. Ainsi, on a recours plus particulièrement aux catalyseurs suivants : tétraborate de sodium ou de potassium, acide orthoborique ; oxyde d'étain, stannate de sodium ou de potassium ; carbonate de bismuth, de césium ou de rubidium ; oxyde de molybdène, d'aluminium, d'indium ou d'antimoine ; acétate de thallium.

Conformément au procédé de l'invention, on met en contact l'acide dicarboxylique avec l'un des catalyseurs précités. L'invention n'exclut pas les mélanges de catalyseurs.

Les catalyseurs de l'invention étant actifs à basse température, le procédé de l'invention est mis en oeuvre en phase liquide, de préférence, en présence d'un solvant réactionnel.

L'acide dicarboxylique peut être utilisé en tant que solvant réactionnel mais d'une manière préférentielle, on fait appel à un solvant organique en tant que flux de transfert thermique.

Plusieurs impératifs président au choix du solvant organique.

Il doit être stable dans les conditions réactionnelles et inerte vis-à-vis de l'acide dicarboxylique de départ et de la cétone cyclique obtenue.

Il doit présenter une température d'ébullition élevée, de préférence comprise entre 200°C et 500°C.

Comme exemples de solvants convenant particulièrement à l'invention, on peut mentionner entre autres :

- les hydrocarbures aliphatiques et/ou aromatiques et plus particulièrement les paraffines tels que notamment, le décane, le undécane, le dodécane ou le tétradécane ; les hydrocarbures aromatiques comme notamment les xylènes, le cumène, les coupes pétrolières constituées de mélange d'alkylbenzènes notamment les coupes de type Solvesso®,
- les esters lourds d'acides minéraux (par exemple, le phosphate de tricrésyle) ou d'acides carboxyliques (par exemple, le phtalate d'octyle),
- les éthers et plus particulièrement les éthers aromatiques comme l'oxyde de biphényle et/ou l'oxyde de benzyle,
- les huiles paraffiniques et/ou naphténiques, résidus de distillation du pétrole.

On peut également mettre en oeuvre un mélange de solvants organiques.

Interviennent donc dans le procédé de l'invention, l'acide dicarboxylique de départ, le catalyseur de la réaction et le solvant organique.

La concentration de l'acide dicarboxylique dans la masse réactionnelle peut varier largement. Généralement, l'acide dicarboxylique représente de 20 à 50 % du poids de la masse réactionnelle.

La quantité de catalyseur mise en oeuvre, exprimée en nombre d'atomes de cation métallique pour 100 moles d'acide dicarboxylique peut être avantageusement comprise entre 0,1 et 20 %, de préférence entre 1 et 10 %.

D'un point de vue pratique, lorsque le procédé de l'invention est mis en oeuvre en discontinu, généralement, on charge d'abord le solvant réactionnel et le catalyseur puis l'on ajoute l'acide dicarboxylique, de préférence fondu préalablement.

Le procédé de l'invention peut être mis en oeuvre aussi bien en discontinu qu'en continu. Dans ce cas-là, seul est alimenté l'acide dicarboxylique.

Le débit d'alimentation de l'acide dicarboxylique peut varier largement, entre 0,1 et 4,0 kg/heure pour un kilogramme de catalyseur introduit. Il est choisi préférentiellement entre 0,5 et 1,0 kg/heure et par kilogramme de catalyseur.

Le procédé de l'invention est avantageusement conduit à une température inférieure à 300°C, de préférence comprise entre 200°C et 300°C et encore plus préférentiellement entre 250°C et 290°C.

Il est généralement mis en oeuvre sous pression atmosphérique mais également sous pression réduite comprise par exemple entre 50 et 760 mm de mercure.

Une variante préférée du procédé de l'invention consiste à éliminer par distillation, au fur et à mesure de leur formation, la cétone cyclique, le gaz carbonique et l'eau formés.

En fin de réaction, on récupère la cétone cyclique à partir du distillat, selon les techniques classiques utilisées dans ce domaine, notamment par décantation ou par cristallisation.

Le procédé de l'invention est tout à fait bien adapté à la préparation de la cyclopentanone, de la diméthyl-2,2 cyclopentanone et de la cyclohexanone.

Les exemples qui suivent, illustrent l'invention sans toutefois la limiter. Dans les exemples, les abréviations suivantes signifient :

RAH = rapport d'alimentation horaire en acide adipique par rapport au catalyseur.

$$RAH = \frac{\text{charge horaire en acide adipique (en poids)}}{\text{charge en catalyseur (en poids)}}$$

$$TT = \frac{\text{nombre de moles d'acide adipique transformées}}{\text{nombre de moles d'acide adipique introduites}}\%$$

$$RT = \frac{\text{nombre de moles de cyclopentanone formées}}{\text{nombre de moles d'acide adipique transformées}}\%$$

<u>EXEMPLES</u>

<u>Exemples 1 à 10 :</u>

Les exemples suivants sont conduits selon un mode de réalisation en continu.

On donne, ci-après, le protocole opératoire qui va être suivi dans tous les exemples.

L'appareillage utilisé est toujours le même. Il s'agit d'un ballon en verre de 1000 ml, muni d'une agitation magnétique, surmonté d'une colonne Raschig de 20 mm de diamètre et de 100 mm de hauteur. La tête de colonne comprend une ampoule de coulée chauffée avec un pistolet à air chaud pour alimenter l'acide adipique qui est préalablement fondu.

On charge le solvant réactionnel et le catalyseur puis l'on ajoute l'acide adipique fondu.

On effectue une distillation en continu de la cyclopentanone à 130°C en tête, en alimentant l'acide adipique sur le mélange solvant/catalyseur maintenu à une température de 250°C.

Les différentes quantités d'acide adipique et de catalyseur chargées sont indiquées dans le tableau récapitulatif qui suit.

La nature du solvant réactionnel est précisée dans ledit tableau. Le volume de solvant engagé est de 500ml. On précisera que le Nyflex 810 mis en oeuvre dans l'exemple 7, est un mélange d'hydrocarbures aromatiques (10 %), naphténiques (48 %) et paraffiniques (42 %).

En fin de réaction, on récupère un distillat comprenant de l'eau et la cyclopentanone. On sépare l'eau de la cyclopentanone en saturant le distillat par du chlorure de sodium. La cyclopentanone est dosée par chromatographie en phase gazeuse.

Quant au culot de distillation, il est extrait par 3 x 600 ml d'un mélange d'eau et d'hydroxyde de sodium (60/40 en volume). Le volume total est ajusté à 2000 ml. On prélève 10 ml ajusté à 100 ml avec un mélange eau/hydroxyde de sodium (15/85 en volume) à 0,035 % d'acide phosphorique. On dose l'acide adipique non transformé, par chromatographie liquide, haute performance.

Les essais sont conduits selon le protocole opératoire précédemment défini.

Toutes les conditions opératoires et résultats obtenus sont consignés dans le tableau (I) suivant :

Tableau (I)

| Ref. | Solvant | | Catalyseur | | Acide adipique chargé | | RAH | Temps (h) | Acide restant (g) | Cyclo-pentanone | RT (%) |
|---|---|---|---|---|---|---|---|---|---|---|---|
| | Nature | Volume (ml) | Nature | Poids (g) | Total (g) | /h | | | | Poids (g) | |
| 1 | oxyde de biphényle<br>oxyde de benzyle | 450<br>50 | $Na_2B_4O_7$ | 8,08 | 417 | 17,4 | 2,15 | 16 | 21 | 208,5 | 91,5 |
| 2 | dodécane<br>tétra-décane | 350<br>150 | $Na_2B_4O_7$ | 8,1 | 149 | 9,3 | 1,15 | 16 | 84 | 26,2 | 70 |
| 3 | oxyde de biphényle<br>oxyde de benzyle | 450<br>50 | $H_3BO_3$ | 10,6 | 237,1 | 23,7 | 2,37 | 10 | 0,7 | 87,3 | 64,2 |
| 4 | oxyde de biphényle<br>oxyde de benzyle | 450<br>50 | $Ga_2O_3$ | 10 | 297 | 18,6 | 1,86 | 16 | 69 | 99,5 | 75,7 |

| | | | | | | | | | | | |
|---|---|---|---|---|---|---|---|---|---|---|---|
| 5 | oxyde de biphényle<br>oxyde de benzyle | 450<br>50 | Sn | 9,5 | 117,2 | 7,3 | 0,77 | 16 | 17,7 | 46 | 80,3 |
| 6 | oxyde de biphényle<br>oxyde de benzyle | 450<br>50 | SnO | 10,8 | 413,8 | 34,5 | 3,2 | 12 | 0 | 199,1 | 83,6 |
| 7 | Nyflex 810 | 460 | SnO | 10,8 | 414,2 | 25,9 | 2,4 | 16 | 0 | 146,7 | 61,5 |
| 8 | dodécane<br>tétra-décane | 300<br>200 | SnO | 10,8 | 198,2 | 12,4 | 1,15 | 16 | 71,2 | 45,6 | 62,4 |
| 10 | oxyde de biphényle<br>oxyde de benzyle | 450<br>50 | $(BiO)_2CO_3$ | 8 | 200 | 12,5 | 1,56 | 16 | 116 | 33,7 | 70,0 |

Exemples 11 à 18 :

On réalise une série d'essais en discontinu.

Dans un réacteur de 250 ml muni d'une agitation magnétique et surmonté d'une colonne garnie Multiknit de 20 mm de diamètre et 100 mm de hauteur, calorifugée et équipée d'une tête de colonne, on charge :

- le solvant réactionnel qui est l'oxyde de biphényle à raison de 140 ml,
- le catalyseur dont la nature est précisée dans le tableau (II) à raison de 0,01 mol,
- l'acide adipique fondu, soit 0,2 mol (29,2 g).

On porte le mélange réactionnel, à léger reflux de façon que les vapeurs ne dépassent pas le bas de la colonne.

La cyclopentanone formée est distillée en tête de colonne à une température égale à 130°C jusqu'à épuisement.

La durée de l'opération est de 8 heures.

Les résultats obtenus sont rassemblés dans le tableau (II).

Tableau (II)

| Ref. | Catalyseur | | TT (%) | RT (%) |
|---|---|---|---|---|
| | Nature | Poids | | |
| 11 | $Rb_2CO_3$ | 2,31 | 78 | 81 |
| 12 | $Cs_2CO_3$ | 1,68 | 90 | 92 |
| 13 | $NaVO_3$ | 1,22 | 42 | 61 |
| 14 | $MoO_3$ | 1,44 | 87 | 88 |
| 15 | $Al_2O_3$ | 1,02 | 65 | 73 |
| 16 | $In_2O_3$ | 2,77 | 77 | 83 |
| 17 | $Tl(CH_3COO)$ | 2,63 | 69 | 78 |
| 18 | $Sb_2O_3$ | 2,91 | 45 | 66 |

Exemple 19 :

On chauffe 5 g d'acide diméthyl-2,2 adipique, en présence de 1,09 g de borax $Na_2B_4O_7$ et on ajoute 17 ml d'oxyde de biphényle à 225°C en 1 heure 35.

Le distillat refroidi vers 0°C, est biphasique. Après élimination de l'eau par absorption sur sulfate de sodium, la diméthyl-2,2 cyclopentanone obtenue est dosée par chromatographie en phase gazeuse.

Le résidu de chaudière est traité par des lavages aqueux basiques (3 x 100 ml d'une solution aqueuse de soude 1 N) pour extraire l'acide non transformé.

Le dosage, par chromatographie liquide haute performance, conduit aux résultats suivants :

- $TT_{\text{acide adipique}}$ = 87 %,
- $RT_{\text{diméthyl-2,2 cyclopentanone}}$ = 92 %.

**Revendications**

1. Procédé de préparation d'une cétone cyclique à partir d'un acide dicarboxylique répondant à la formule (I) suivante :

$$HOOC - R - COOH \quad (I)$$

dans ladite formule (I), R représente un radical alcoylène, substitué ou non, comprenant un enchaînement linéaire d'atomes en un nombre suffisant pour former le cycle cétonique désiré, deux atomes de carbone vicinaux de l'enchaînement du radical R pouvant faire partie d'un cycle carbocylique ou hététocyclique, saturé ou aromatique. caractérisé par le fait que l'on conduit la réaction en phase liquide, en présence d'une quantité efficace d'un métal ou dérivé des éléments choisis parmi : le bore, l'aluminium, le gallium, l'indium, le thallium, l'étain, l'antimoine, le

bismuth, le molybdène, le rubidium, le césium, le vanadium, à l'exception des phosphates neutres.

2. Procédé selon la revendication 1 caractérisé par le fait que l'acide dicarboxylique mis en oeuvre répond à la formule (I) dans laquelle le radical R comprend un enchaînement linéaire de 2 à 10 atomes et, de préférence, de 2 à 7 atomes et encore plus préférentiellement de 4 ou 5.

3. Procédé selon l'une des revendications 1 et 2 caractérisé par le fait que l'acide dicarboxylique mis en oeuvre répond à la formule (I) dans laquelle le radical R divalent est substitué de telle sorte que la chaîne hydrocarbonée porte des chaînes latérales ou ramifications qui sont constituées par des radicaux alkyle ayant de 1 à 4 atomes de carbone ; lesdites ramifications se situant sur un ou les deux atomes de carbone en position $\alpha$ ou $\beta$ des groupes carboxyliques.

4. Procédé selon l'une des revendications 1 à 3 caractérisé par le fait que l'acide dicarboxylique mis en oeuvre répond à la formule (I) dans laquelle le radical R présente une condensation totale en carbone variant de 2 à 40 atomes de carbone et comprend un enchaînement linéaire de 2 à 10 atomes qui intervient ensuite dans le cycle obtenu.

5. Procédé selon l'une des revendications 1 à 4 caractérisé par le fait que l'acide dicarboxylique mis en oeuvre répond à la formule (I) dans laquelle le radical R représente un radical alcoylène, linéaire ou ramifié ayant de 2 à 12 atomes de carbone qui comprend un enchaînement linéaire de 2 à 8 atomes de carbone entre les deux groupes COOH et, de préférence, de 4 à 5 atomes de carbone entre les deux groupes COOH.

6. Procédé selon l'une des revendications 1 à 5 caractérisé par le fait que l'acide dicarboxylique mis en oeuvre répond à la formule (I) dans laquelle deux atomes de carbone vicinaux de l'enchaînement du radical R font partie d'un cycle carbocyclique ou hététocyclique, saturé ou aromatique.

7. Procédé selon l'une des revendications 1 à 6 caractérisé par le fait que l'acide dicarboxylique de formule (I) mis en oeuvre est choisi parmi :

- l'acide adipique,
- l'acide méthyl-2 adipique
- l'acide méthyl-3 adipique
- l'acide méthyl-4 adipique
- l'acide méthyl-5 adipique
- l'acide diméthyl-2,2 adipique
- l'acide diméthyl-3,3 adipique
- l'acide triméthyl-2,2,5 adipique
- l'acide diméthyl-2,5 adipique
- l'acide pimélique (heptanedioïque)
- l'acide méthyl-2 pimélique
- l'acide diméthyl-2,2 pimélique
- l'acide diméthyl-3,3 pimélique
- l'acide diméthyl-2,5 pimélique
- l'acide triméthyl-2,2,5 pimélique
- l'acide azélaïque
- l'acide sébacique
- l'acide phénylène-1,2 diacétique

8. Procédé selon l'une des revendications 1 à 7 caractérisé par le fait que l'acide dicarboxylique de formule (I) mis en oeuvre est l'acide adipique ou l'acide diméthyl-2,2 adipique.

9. Procédé selon l'une des revendications 1 à 8 caractérisé par le fait que le catalyseur mis en oeuvre est un catalyseur à base des éléments précités : le bore, l'aluminium, le gallium, l'indium, le thallium, l'étain, l'antimoine, le bismuth, le molybdène, le rubidium, le césium, le vanadium, sous forme de métal, d'oxyde ou d'hydroxyde, ou sous forme saline, sel simple ou double, minéral ou organique.

10. Procédé selon l'une des revendications 1 à 9 caractérisé par le fait que le catalyseur mis en oeuvre est un catalyseur à base de bore choisi parmi : les acides boriques, de préférence l'acide orthoborique (ou son précurseur $B_2O_3$), l'acide métaborique, l'acide pyro- ou tétraborique ; les borates métalliques, notamment de métaux alcalins,

alcalino-terreux ou d'ammonium sous forme anhydre ou hydratée, en particulier aux tiers borates, hémiborates, monoborates, diborates, triborates, tétraborates, pentaborates de métaux, de préférence alcalins ou d'ammonium ; les sels doubles contenant du bore, notamment les fluoborates métalliques.

**11.** Procédé selon l'une des revendications 1 à 10 caractérisé par le fait que les éléments métalliques intervenant dans le catalyseur sont apportés sous forme d'un métal ou sous forme d'un oxyde ou d'un hydroxyde ; sous forme d'un sel minéral de préférence, nitrate, sulfate, oxysulfate, halogénure, oxyhalogénure, silicate, carbonate, oxalate ; d'un sel organique de préférence, acétylacétonate, alcoolate et encore plus préférentiellement méthylate ou éthylate, carboxylate et encore plus préférentiellement acétate.

**12.** Procédé selon l'une des revendications 1 à 11 caractérisé par le fait que le catalyseur est choisi parmi les catalyseurs suivants : tétraborate de sodium ou de potassium, acide orthoborique ; oxyde d'étain, stannate de sodium ou de potassium ; carbonate de bismuth, de césium ou de rubidium ; oxyde de molybdène, d'aluminium, d'indium ou d'antimoine ; acétate de thallium.

**13.** Procédé selon l'une des revendications 1 à 12 caractérisé par le fait que la réaction est conduite en phase liquide, en présence d'un solvant réactionnel.

**14.** Procédé selon la revendication 13 caractérisé par le fait que le solvant réactionnel est choisi parmi :

- les hydrocarbures aliphatiques et/ou aromatiques et plus particulièrement les paraffines tels que notamment, le décane, le undécane, le dodécane ou le tétradécane ; les hydrocarbures aromatiques comme notamment les xylènes, le cumène, les coupes pétrolières constituées de mélange d'alkylbenzènes notamment les coupes de type Solvesso®,
- les esters lourds d'acides minéraux (par exemple, le phosphate de tricrésyle) ou d'acides carboxyliques (par exemple, le phtalate d'octyle),
- les éthers et plus particulièrement les éthers aromatiques comme l'oxyde de biphényle et/ou l'oxyde de benzyle,
- les huiles paraffiniques et/ou naphténiques, résidus de distillation du pétrole.

**15.** Procédé selon l'une des revendications 1 à 14 caractérisé par le fait que la concentration de l'acide dicarboxylique dans la masse réactionnelle représente de 20 à 50 % du poids de la masse réactionnelle.

**16.** Procédé selon l'une des revendications 1 à 15 caractérisé par le fait que la quantité de catalyseur mise en oeuvre, exprimée en nombre d'atomes de cation métallique pour 100 moles d'acide dicarboxylique est comprise entre 0,1 et 20 %, de préférence entre 1 et 10 %.

**17.** Procédé selon l'une des revendications 1 à 16 caractérisé par le fait que l'on charge d'abord le solvant réactionnel et le catalyseur puis l'on ajoute l'acide dicarboxylique, de préférence fondu préalablement.

**18.** Procédé selon l'une des revendications 1 à 17 caractérisé par le fait que le débit d'alimentation de l'acide dicarboxylique varie entre 0,1 et 4,0 kg/heure pour un kilogramme de catalyseur introduit ; de préférence, entre 0,5 et 1,0 kg/heure et par kilogramme de catalyseur.

**19.** Procédé selon l'une des revendications 1 à 18 caractérisé par le fait que la température réactionnelle est comprise entre 200 et 300°C, de préférence entre 250°C et 290°C.

## Claims

**1.** A process for the preparation of a cyclic ketone from a dicarboxylic acid corresponding to the following formula (I):

$$HOOC - R - COOH \qquad (I)$$

in which formula (I), R represents a substituted or unsubstituted alkylene radical comprising a straight chain of atoms in a sufficient number to form the desired ketonic ring, two adjacent carbon atoms of the chain of the radical R being capable of forming part of a saturated or aromatic carbocyclic or heterocyclic ring,
characterised in that the reaction is carried out in liquid phase in the presence of an effective amount of a metal or derivative of the elements selected from : boron, aluminium, gallium, indium, thallium, tin, antimony, bismuth,

molybdenum, rubidium, caesium, vanadium, with the exception of neutral phosphates.

2. A process according to claim 1, characterised in that the dicarboxylic acid used corresponds to the formula (I) in which the radical R comprises a straight chain of from 2 to 10 atoms, and preferably from 2 to 7 atoms, and still more preferably 4 or 5 atoms.

3. A process according to one of claim 1 and claim 2, characterised in that the dicarboxylic acid used corresponds to the formula (I) in which the divalent radical R is substituted in such a way that the hydrocarbon chain bears lateral chains or branch chains which are formed by alkyl radicals having from 1 to 4 carbon atoms; said branch chains being disposed on one or both the carbon atoms in the position $\alpha$ or $\beta$ of the carboxylic groups.

4. A process according to one of claims 1 to 3, characterised in that the dicarboxylic acid used corresponds to the formula (I), in which the radical R has a total condensation of carbon varying from 2 to 40 carbon atoms and comprises a straight chain of from 2 to 10 atoms which are then involved in the ring produced.

5. A process according to one of claims 1 to 4, characterised in that the dicarboxylic acid used corresponds to the formula (I) in which the radical R represents a straight or branched alkylene radical having from 2 to 12 carbon atoms which comprises a straight chain of from 2 to 8 carbon atoms between the two COOH groups, and, preferably, from 4 to 5 carbon atoms between the two COOH groups.

6. A process according to one of claims 1 to 5, characterised in that the dicarboxylic acid used corresponds to the formula (I) in which two adjacent carbon atoms of the chain of the radical R form part of a saturated or aromatic carbocyclic or heterocyclic ring.

7. A process according to one of claims 1 to 6, characterised in that the dicarboxylic acid of the formula (I) used is selected from:

- adipic acid,
- 2-methyl adipic acid,
- 3-methyl adipic acid,
- 4-methyl adipic acid,
- 5-methyl adipic acid,
- 2,2-dimethyl adipic acid,
- 3,3-dimethyl adipic acid,
- 2,2,5-trimethyl adipic acid,
- 2,5-dimethyl adipic acid,
- pimelic (heptanedioic) acid,
- 2-methyl pimelic acid,
- 2,2-dimethyl pimelic acid,
- 3,3-dimethyl pimelic acid,
- 2,5-dimethyl pimelic acid,
- 2,2,5-trimethyl pimelic acid,
- azelaic acid,
- sebacic acid,
- 1,2-phenylene diacetic acid.

8. A process according to one of claims 1 to 7, characterised in that the dicarboxylic acid of formula (I) used is adipic acid or 2,2-dimethyl adipic acid.

9. A process according to one of claims 1 to 8, characterised in that the catalyst used is a catalyst based on the above-mentioned elements : boron, aluminium, gallium, indium, thallium, tin, antimony, bismuth, molybdenum, rubidium, caesium, vanadium, in the form of metal, oxide or hydroxide, or in saline form, simple or double salt, mineral or organic.

10. A process according to one of claims 1 to 9, characterised in that the catalyst used is a catalyst based on boron, selected from : boric acids, preferably orthoboric acid (or its precursor $B_2O_3$), metaboric acid, pyro- or tetraboric acid; metallic borates, in particular of alkali metals or alkaline-earth metals or ammonium, in anhydrous or hydrated form, in particular with third borates, hemiborates, monoborates, diborates, triborates, tetraborates, pentaborates

of metals, preferably alkali or ammonium; double salts containing boron, in particular metallic fluoborates.

**11.** A process according to one of claims 1 to 10, characterised in that the metallic elements involved in the catalyst are provided in the form of a metal or in the form of an oxide or a hydroxide; in the form of a mineral salt, preferably nitrate, sulphate, oxysulphate, halide, oxyhalide, silicate, carbonate, oxalate; of an organic salt, preferably acetylacetonate, alcoholate, and still more preferably methylate or ethylate, carboxylate, and still more preferably, acetate.

**12.** A process according to one of claims 1 to 11, characterised in that the catalyst is selected from the following catalysts: sodium or potassium tetraborate, orthoboric acid; tin oxide, sodium or potassium stannate; bismuth, caesium or rubidium carbonate; molybdenum, aluminium, indium or antimony oxide; thallium acetate.

**13.** A process according to one of claims 1 to 12, characterised in that the reaction is carried out in liquid phase, in the presence of a reaction solvent.

**14.** A process according to claim 13, characterised in that the reaction solvent is selected from:

- aliphatic and/or aromatic hydrocarbons, and, more particularly, paraffins such as, in particular, decane, undecane, dodecane or tetradecane; aromatic hydrocarbons, such as, in particular, xylenes, cumene, petroleum cuts formed by a mixture of alkyl benzenes, in particular cuts of Solvesso® type,
- heavy esters of mineral acids (e.g. tricresyl phosphate) or carboxylic acids (e.g. octyl phthalate),
- ethers, and, more particularly, aromatic ethers, such as biphenyl oxide and/or benzyl oxide, and
- paraffinic and/or naphthenic oils, petroleum distillation residues.

**15.** A process according to one of claims 1 to 14, characterised in that the concentration of dicarboxylic acid in the reaction mass represents from 20 to 50% of the weight of the reaction mass.

**16.** A process according to one of claims 1 to 15, characterised in that the quantity of catalyst used, expressed in terms of the number of atoms of metallic cation for 100 moles of dicarboxylic acid, is between 0.1 and 20%, preferably between 1 and 10%.

**17.** A process according to one of claims 1 to 16, characterised in that the reaction solvent and the catalyst are introduced first of all, and the dicarboxylic acid which has preferably been melted beforehand is added next.

**18.** A process according to one of claims 1 to 17, characterised in that the dicarboxylic acid feed flow rate varies between 0.1 and 4.0 kg/hour for one kilogram of catalyst introduced; preferably between 0.5 and 1.0 kg/hour and per kilogram of catalyst.

**19.** A process according to one of claims 1 to 18, characterised in that the reaction temperature is between 200 and 300°C, preferably between 250°C and 290°C.

**Patentansprüche**

**1.** Verfahren zur Herstellung eines cyclischen Ketons ausgehend von einer Dicarbonsäure der folgenden Formel (I)

$$HOOC\text{-}R\text{-}COOH \quad (I)$$

in der R einen gegebenenfalls substituierten Alkylenrest darstellt, der eine lineare Kette von Atomen in einer Anzahl umfaßt, die ausreicht, um den gewünschten Ketonring auszubilden, wobei zwei benachbarte Kohlenstoffatome der Kette des Rests R Teil eines gesättigten oder aromatischen, carbocyclischen oder heterocyclischen Rings sein können,
dadurch gekennzeichnet, daß man die Reaktion in flüssiger Phase in Gegenwart einer ausreichenden Menge eines Metalls oder eines Derivates von Elementen durchführt, ausgewählt aus Bor, Aluminium, Gallium, Indium, Thallium, Zinn, Antimon, Wismut, Molybdän, Rubidium, Cäsium und Vanadium, mit Ausnahme der neutralen Phosphate.

**2.** Verfahren nach Anspruch 1, dadurch gekennzeichnet, daß die eingesetzte Dicarbonsäure der Formel (I) entspricht, in der der Rest R eine lineare Kette mit 2 bis 10 Atomen, vorzugsweise mit 2 bis 7 Atomen, insbesondere mit 4 oder 5 Atomen, darstellt.

3. Verfahren nach einem der Ansprüche 1 und 2, dadurch gekennzeichnet, daß die eingesetzte Dicarbonsäure der Formel (I) entspricht, in der der zweiwertige Rest R derart substituiert ist, daß die Kohlenwasserstoffkette Seitenketten oder Verzweigungen enthält, die aus Alkylresten mit 1 bis 4 Kohlenstoffatomen bestehen, wobei sich diese Verzweigungen an einem oder an den beiden Kohlenstoffatomen in $\alpha$- oder $\beta$-Position zu den Carboxylgruppen befinden.

4. Verfahren nach einem der Ansprüche 1 bis 3, dadurch gekennzeichnet, daß die eingesetzte Dicarbonsäure der Formel (I) entspricht, in der der Rest R ein Gesamtkondensation in bezug auf Kohlenstoff von 2 bis 40 Kohlenstoffatomen aufweist und eine lineare Kette mit 2 bis 10 Atomen umfaßt, die sich in dem erhaltenen Ring befindet.

5. Verfahren nach einem der Ansprüche 1 bis 4, dadurch gekennzeichnet, daß die eingesetzte Dicarbonsäure der Formel (I) entspricht, in der der Rest R einen linearen oder verzweigten Alkylenrest mit 2 bis 12 Kohlenstoffatomen darstellt, der eine lineare Kette mit 2 bis 8 Kohlenstoffatomen zwischen den zwei COOH-Gruppen, vorzugsweise mit 4 bis 5 Kohlenstoffatomen zwischen den COOH-Gruppen umfaßt.

6. Verfahren nach einem der Ansprüche 1 bis 5, dadurch gekennzeichnet, daß die eingesetzte Dicarbonsäure der Formel (I) entspricht, in der zwei benachbarte Kohlenstoffatome der Kette des Rests R zu einem gesättigten oder aromatischen, heterocyclischen oder carbocyclischen Ring gehören.

7. Verfahren nach einem der Ansprüche 1 bis 6, dadurch gekennzeichnet, daß die eingesetzte Dicarbonsäure der Formel (I) ausgewählt ist aus:

- Adipinsäure,
- 2-Methyladipinsäure,
- 3-Methyladipinsäure,
- 4-Methyladipinsäure,
- 5-Methyladipinsäure,
- 2,2-Dimethyladipinsäure,
- 3,3-Dimethyladipinsäure,
- 2,2,5-Trimethyladipinsäure,
- 2,5-Dimethyladipinsäure,
- Pimelinsäure (Heptandisäure),
- 2-Methylpimelinsäure,
- 2,2-Dimethylpimelinsäure,
- 3,3-Dimethylpimelinsäure,
- 2,5-Dimethylpimelinsäure,
- 2,2,5-Trimethylpimelinsäure,
- Azelainsäure
- Sebacinsäure,
- 1,2-Phenylendiessigsäure.

8. Verfahren nach einem der Ansprüche 1 bis 7, dadurch gekennzeichnet, daß die eingesetzte Dicarbonsäure der Formel (I) Adipinsäure oder 2,2-Dimethyladipinsäure ist.

9. Verfahren nach einem der Ansprüche 1 bis 8, dadurch gekennzeichnet, daß der eingesetzte Katalysator ein Katalysator auf Basis der zuvor genannten Elemente Bor, Aluminium, Gallium, Indium, Thallium, Zinn, Antimon, Wismut, Molybdän, Rubidium, Cäsium und Vanadium in Metall-, Oxid- oder Hydroxydform oder in Form eines einfachen oder doppelten, anorganischen oder organischen Salzes ist.

10. Verfahren nach einem der Ansprüche 1 bis 9, dadurch gekennzeichnet, daß der eingesetzte Katalysator ein Katalysator auf Basis von Bor ist, ausgewählt aus Borsäuren, vorzugsweise Orthoborsäure (oder ihrem Vorläufer $B_2O_3$), Metaborsäure, Pyroborsäure oder Tetraborsäure; Metallboraten, insbesondere Boraten von Alkalimetallen, Erdalkalimetallen oder Ammonium in wasserfreier oder hydratisierter Form, insbesondere Drittelboraten, Hemiboraten (Halbboraten), Monoboraten, Diboraten, Triboraten, Tetraboraten und Pentaboraten von Metallen, vorzugsweise von Alkalimetallen oder Ammonium; borhaltigen Doppelsalzen, insbesondere Metallfluoroboraten.

11. Verfahren nach einem der Ansprüche 1 bis 10, dadurch gekennzeichnet, daß die in dem Katalysator vorhandenen Metallelemente eingebracht werden in Metallform oder in Form eines Oxids oder Hydroxids; in Form eines anorganischen Salzes, vorzugsweise eines Nitrats, Sulfats, Oxysulfats, Halogenids, Oxyhalogenids, Silikats, Carbonats oder Oxalats; in Form eines organischen Salzes, vorzugsweise eines Acetylacetonats, Alkoholats, insbesondere eines Methylats oder Ethylats, ganz besonders eines Acetats.

**12.** Verfahren nach einem der Ansprüche 1 bis 11, dadurch gekennzeichnet, daß der Katalysator ausgewählt ist aus den folgenden Katalysatoren: Natrium- oder Kaliumtetraborat, Orthoborsäure; Zinnoxid, Natriumstannat oder Kaliumstannat; Wismut-, Cäsium- oder Rubidiumcarbonat; Molybdän-, Aluminium-, Indium- oder Antimonoxid; Thalliumacetat.

**13.** Verfahren nach einem der Ansprüche 1 bis 12, dadurch gekennzeichnet, daß die Reaktion in flüssiger Phase in Gegenwart eines Reaktionslösungsmittels durchgeführt wird.

**14.** Verfahren nach Anspruch 13, dadurch gekennzeichnet, daß das Reaktionslösungsmittel ausgewählt ist aus:

- aliphatischen und/oder aromatischen Kohlenwasserstoffen, insbesondere Paraffinen, wie vor allem Dekan, Undekan, Dodekan oder Tetradekan; aromatischen Kohlenwasserstoffen, wie insbesondere Xylolen, Kumol und Kohlenwasserstofffraktionen, die aus einer Mischung von Alkylbenzolen bestehen, insbesondere Fraktionen vom Solvesso®-Typ,
- schweren Estern von anorganischen Säuren (z.B. Trikresylphosphat) oder Carbonsäuren (z.B. Octylphthalat),
- Ethern, insbesondere aromatischen Ethern, wie Biphenyloxid und/oder Benzyloxid,
- Paraffinölen und/oder Naphtenölen, Rückständen aus der Erdöldestillation.

**15.** Verfahren nach einem der Ansprüche 1 bis 14, dadurch gekennzeichnet, daß die Konzentration der Dicarbonsäure in der Reaktionsmasse 20 bis 50 Gew.-% der Reaktionsmasse darstellt.

**16.** Verfahren nach einem der Ansprüche 1 bis 15, dadurch gekennzeichnet, daß die eingesetzte Katalysatormenge, berechnet als Anzahl der Metallkationatome auf 100 Mol Dicarbonsäure, zwischen 0,1 und 20 %, vorzugsweise zwischen 1 und 10 %, liegt.

**17.** Verfahren nach einem der Ansprüche 1 bis 16, dadurch gekennzeichnet, daß man zunächst das Reaktionslösungsmittel und den Katalysator einbringt und anschließend die Dicarbonsäure hinzugibt, die vorzugsweise zuvor aufgeschmolzen worden ist.

**18.** Verfahren nach einem der Ansprüche 1 bis 17, dadurch gekennzeichnet, daß der Zufuhrdurchsatz an Dicarbonsäure zwischen 0,1 und 4,0 kg/Stunde pro ein Kilogramm des zugegebenen Katalysators, vorzugsweise zwischen 0,5 und 1,0 kg/Stunde pro Kilogramm des Katalysators, variiert.

**19.** Verfahren nach einem der Ansprüche 1 bis 18, dadurch gekennzeichnet, daß die Reaktionstemperatur zwischen 200 und 300 °C, vorzugsweise zwischen 250 und 290 °C, liegt.